# FASCICULE DE BREVET EUROPEEN

(11) **EP 0 908 196 B1**
(45) Date de publication et mention de la délivrance du brevet: **12.01.2005**
(21) Numéro de dépôt: 98402482.8
(22) Date de dépôt: 07.10.1998
(51) Int. Cl.: A61N 1/37, A61B 5/0452

(54) **Stimulateur / défibrillateur cardiaque , comprenant des moyens de détection et de détermination de l'instant des dépolarisations spontanées du myocarde**
Herzschrittmacher / Defibrillator mit Detektor für spontane Myokardiumdepolarisationen und deren Entstehungszeit
Pacemaker / defibrillator with means to detect the occurrence and timing of spontaneous depolarisations of the myocardium

(30) Priorité: 07.10.1997 FR 9712463
(43) Date de publication de la demande: 14.04.1999
(73) Titulaire: ELA MEDICAL (Société anonyme), 92541 Montrouge (FR)
(72) Inventeur: Bonnet, Jean-Luc, 92120 Montrouge (FR); Nitzsche, Rémi, 78950 Gambais (FR)
(74) Mandataire: Dupuis-Latour, Dominique

(56) Documents cités:
- EP-A- 0 321 764
- EP-A- 0 554 208
- US-A- 5 273 049
- SIMPSON E V ET AL: "AN AUTOMATIC ACTIVATION DETECTOR FOR BIPOLAR CARDIAC ELECTROGRAMS" CARDIOLOGY AND IMAGING, NEW ORLEANS, NOV. 4 - 7, 1988, vol. VOL. 1, no. CONF. 10, 4 novembre 1988, page 113/114 XP000093888 HARRIS G;WALKER C
- KIRHNER M ET AL: "REAL-TIME INTERVAL MEASUREMENT DURING INVASIVE CARDIAC ELECTROPHYSIOLOGIC TESTING" PROCEEDINGS OF THE COMPUTERS IN CARDIOLOGY MEETING, LEUVEN, SEPT. 12 - 15, 1987, no. MEETING 14, 12 septembre 1987, pages 93-96, XP000094146 RIPLEY K L

## Description

L'invention concerne les "dispositifs médicaux implantables actifs" tels que définis par la directive 90/385/CEE du 20 juin 1990 du Conseil des communautés européennes, et plus précisément les dispositifs stimulateurs cardiaques, défibrillateurs et/ou cardioverteurs qui comportent une fonction de détection des dépolarisations spontanées du myocarde.

Ces dispositifs détectent et analysent différents signaux émis par le myocarde et constitués de différentes ondes. Diverses fonctions de ces appareils nécessitent la détection de l'onde de dépolarisation, révélatrice d'une activité spontanée des cellules myocardiques, qu'il s'agisse de la cavité auriculaire ou de la cavité ventriculaire.

La détection d'une telle onde de dépolarisation permet tout d'abord de réinitialiser certaines commandes temporelles du dispositif, telles que l'intervalle d'échappement, ou d'en déclencher d'autres, telles que le délai auriculo-ventriculaire.

Cette détection est également utilisée pour commander le fonctionnement de certains algorithmes tels que, d'une part, les algorithmes de repli ou *fallback,* de lissage, etc. D'autre part, la détection du rythme ventriculaire spontané, notamment l'analyse de sa stabilité, est dans certains défibrillateurs implantables un paramètre essentiel de déclenchement de la thérapie de choc.

Enfin, certains dispositifs sont équipés de fonctions mémoire dites "Holter" permettant un suivi continu des paramètres du rythme cardiaque, en particulier pour évaluer la stabilité de celui-ci à des fins diagnostiques et/ou thérapeutiques.

Les systèmes connus de détection des ondes de dépolarisation utilisent divers moyens, les plus fréquents utilisant, comme décrit par exemple dans le EP-A-0 605 264 (Ela Médical), un filtrage du signal myocardique dans la gamme des fréquences recherchée, par exemple 30 à 80 Hz, le signal filtré étant ensuite appliqué à un circuit à seuil, unilatéral ou bilatéral, pour la détection de l'onde de dépolarisation.

On connaît également, par exemple d'après le EP-A-0 775 502 (Ela Médical) des systèmes plus élaborés, basés sur l'analyse de la dérivée première (taux d'accroissement) du signal.

Dans tous les cas, il s'agit de détecter le plus rapidement possible et de façon fiable (c'est-à-dire sensible) la présence d'une onde de dépolarisation.

En effet, une telle rapidité de détection est importante, car la présence d'une éventuelle onde de dépolarisation spontanée doit être signalée avant la fin de l'intervalle d'échappement ; dans le cas contraire, une stimulation serait appliquée inutilement. Bien qu'elle ne soit pas dangereuse, il est souhaitable d'éviter une telle stimulation avec l'activité spontanée concurrente (phénomène dit de "fusion"), d'une part pour économiser de l'énergie, d'autre part afin que l'activité spontanée du patient puisse être réellement suivie par le système, par exemple pour des fonctions diagnostiques ou pour des algorithmes prédicteurs d'arythmie basés sur la stabilité du rythme spontané.

Les procédés connus de détection remplissent convenablement les exigences de sensibilité et de rapidité dans la détection de présence d'une onde de dépolarisation spontanée.

Toutefois, ils présentent l'inconvénient de ne pas toujours indiquer, d'un cycle à l'autre, le même instant auquel est détectée l'onde de dépolarisation. En effet, l'onde de dépolarisation comporte plusieurs phases qui varient légèrement d'un cycle à l'autre et l'on peut se trouver en présence, sur certains cycles, d'une légère modification d'amplitude, voire de morphologie. Ainsi les systèmes de détection peuvent détecter alternativement une phase de l'onde ou une autre (par exemple un pic positif ou un pic négatif). De la sorte, un rythme stable (au regard des différentes composantes du signal) pourra être vu instable par le système, avec des variations pouvant être typiquement supérieures à 20 ms. Ceci peut être pénalisant lorsque, précisément, l'objectif est de mesurer certains paramètres temporels du rythme, par exemple à des fins de diagnostic ou pour déterminer le comportement de certains algorithmes.

Le EP-A-0 554 208 divulgue un dispositif implantable comprenant les éléments visés au préambule de la revendication 1, pour analyser le profil de l'onde cardiaque et mesurer certaines durées caractéristiques à des fins de diagnostic, notamment pour détecter des épisodes d'arythmie et/ou d'ischémie.

L'un des buts de l'invention est de proposer un dispositif du type précité comportant des moyens de détection de dépolarisation qui soient à la fois sensibles et rapides - pour délivrer une information de survenue ou non d'une dépolarisation (aspect qualitatif) - et suffisamment spécifiques dans la définition temporelle de l'instant de survenue de la dépolarisation (aspect quantitatif, car il permet la mesure des séquencements temporels de manière que les ondes de même nature soient toujours vues sur la même composante de l'onde par le système).

Ainsi, les temps (par exemple délai entre les ondes de dépolarisation successives) mesurés par le système de détection correspondront véritablement à des caractéristiques propres du rythme.

Le dispositif de l'invention, qui est du type général divulgué par le EP-A-0 554 208, comprend les éléments caractéristiques énoncés par la revendication 1. Les sous-revendications visent des mises en oeuvre particulières at avantageuses.

D'autres caractéristiques et avantages de l'invention apparaîtront à la lecture de la description détaillée ci-dessous d'un exemple de mise en oeuvre, en référence aux dessins annexés.
La figure 1 illustre le fonctionnement de base d'un système selon l'art antérieur de détection des dépolarisations spontanées.
La figure 2 illustre le fonctionnement du système selon l'invention.
La figure 3 est un chronogramme comparatif entre un système selon l'art antérieur et un système selon l'invention.

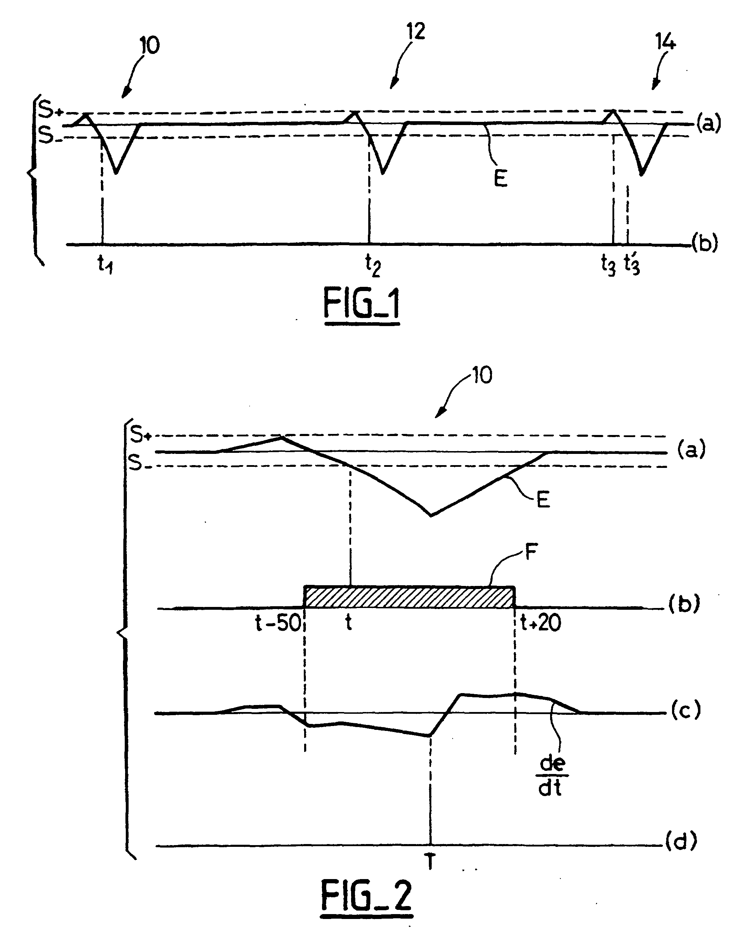

Sur la figure 1, on a représenté trois cycles successifs 10, 12, 14 d'un signal endocavitaire E, résultant de dépolarisations spontanées.

Les systèmes de l'art antérieur utilisent généralement une technique à seuil d'amplitude, c'est-à-dire que l'on considère qu'il y a détection d'une dépolarisation spontanée du myocarde lorsque l'amplitude du signal endocavitaire E dépasse, en valeur absolue, un seuil prédéfini S (S+ ou S-), ce franchissement intervenant aux instants t₁, t₂ et t₃ sur la figure 1 (on notera que cette technique à seuil n'est pas limitative, et que d'autres techniques de détection de la dépolarisation peuvent être envisagées pour mettre en oeuvre la présente invention).

Les tops de détection aux instants t₁, t₂ et t₃ (1°) révèlent la survenue d'une dépolarisation spontanée et (2°) permettent d'évaluer des durées I₁ = t₂-t₁, I₂ = t₃-t₂, etc. représentatives des intervalles séparant deux dépolarisations successives (intervalles R-R).

Il arrive toutefois que le signal endocavitaire E présente certaines irrégularités d'une onde à la suivante, par exemple pour les ondes 10 et 12 le franchissement du seuil a lieu sur le seuil négatif S- tandis que pour l'onde 14, dont l'amplitude du précurseur de l'onde est très légèrement supérieure à ce qu'elle était pour les deux ondes précédentes, cette détection s'effectue sur le seuil positif S+. Ainsi, pour l'onde 14, la détection s'effectuera à un instant t₃ antérieur à l'instant t'₃ homologue des instants t₁ et t₂ (c'est-à-dire le franchissement du seuil négatif S-). Dans ce dernier cas, il n'y a pas fausse détection, seulement détection anticipée. Mais ceci peut avoir une incidence sur l'évaluation de la stabilité du rythme car, même en présence d'un rythme stable (comme dans le cas de la figure 1), le système déterminera un intervalle calculé I₂ = t₃-t₂ plus court que l'intervalle réel t'₃-t₂.

On constate ainsi que les systèmes de détection de l'art antérieur peuvent de temps en temps présenter des anomalies dans le calcul de l'intervalle R-R, et donc dans l'évaluation de la variabilité du rythme sur plusieurs cycles successifs, anomalies ne correspondant pas à une variabilité réelle de ce rythme. En d'autres termes, même en présence d'un rythme stable, le système peut détecter, à tort, une instabilité de ce rythme, due en fait à la variabilité de l'instant de détection de l'onde de dépolarisation d'un cycle au suivant (et non à une variation de la périodicité de ces ondes).

On pourrait certes pallier cet inconvénient en relevant le seuil S, mais cette modification se ferait au détriment de la rapidité de réponse du système, ce qui serait un inconvénient majeur car il est nécessaire de pouvoir déterminer de la manière la plus précoce possible si une onde de dépolarisation est ou non apparue, afin de prendre les mesures nécessaires, notamment pour éviter de déclencher une stimulation inopportune. De plus, une telle élévation du seuil serait préjudiciable à la qualité de la détection, principalement en cas d'arythmie.

La figure 2 illustre le fonctionnement selon l'invention, destiné à supprimer cet inconvénient.

Le principe essentiel de l'invention consiste à prévoir deux systèmes de détection distincts, fonctionnant en cascade.

Le premier système est le système classique, à seuil ou analogue (par exemple celui du EP-A-0 775 502 précité basé sur l'analyse de la dérivée première du signal), que l'on vient de décrire en référence à la figure 1 et illustré sur le chronogramme (a) de la figure 2, qui présente l'avantage de permettre une détection rapide et sensible de la survenue d'une dépolarisation spontanée, à l'instant repéré t sur le chronogramme (b) de la figure 2. Il ne sera, en revanche, pas utilisé pour la mesure des intervalles entre cycles.

Ce paramètre sera évalué par un second système de détection, moins rapide mais plus spécifique.

Les deux systèmes ne travaillent pas en parallèle, mais en cascade, c'est-à-dire que l'on est en présence d'un mécanisme à double détente, le second système (pour l'évaluation de l'intervalle du cycle) n'étant activé que si le premier système (détection d'une dépolarisation spontanée) a révélé la survenue d'une première dépolarisation et n'indique pas la détection d'une suivante.

A ce moment, le second système ouvre une "fenêtre d'analyse", par exemple de l'ordre de 200 ms. Il est possible d'envisager une fenêtre rétrograde supplémentaire de -50 ms : la fenêtre résultante totale serait de [-50 ms, +200 ms] autour de la détection primaire, comme illustré sur le chronogramme (b) de la figure 2. Comme exemple illustratif, le second système pourrait pendant la fenêtre d'analyse" rechercher dans le signal endocavitaire une singularité prédéfinie, la position temporelle de cette singularité définissant l'instant de survenue de la dépolarisation pour l'évaluation de la variabilité du rythme.

La singularité en question pourrait être notamment l'extremum négatif de la dérivée première du signal cardiaque, comme illustré sur le chronogramme (c) de la figure 2 : sur la deuxième ligne de cette figure, on a illustré la dérivée dE/dt du signal endocavitaire E (en pratique, le signal donné par les incréments successifs ΔE du signal cardiaque échantillonné par intervalles discrets Δt de la fréquence d'échantillonnage), qui présente une valeur absolue maximale à l'instant T. Le choix de cette singularité particulière présente l'avantage d'être un critère unique pour une onde donnée. Pour des cycles de même nature, ce critère ne sera pas influencé par des modifications telles que l'amplitude ou la variabilité de la ligne de base.

De façon caractéristique de l'invention, on recherche la caractéristique unique dans le signal cardiaque par une méthode d'autocorrélation.

Le système enregistre pendant la fenêtre de temps des échantillons du signal à fréquence élevée. Lors de la détection suivante à l'aide du moyen primaire, le signal est enregistré de la même façon. Une recherche du pic d'autocorrélation entre informations contenues dans les deux fenêtres permet alors de déterminer le moment T où la dépolarisation au sens de l'invention est survenue. Le décalage dans la superposition des fenêtres donne le décalage du troisième cycle cardiaque des figures 1 et 2 (t'₃-t₃).

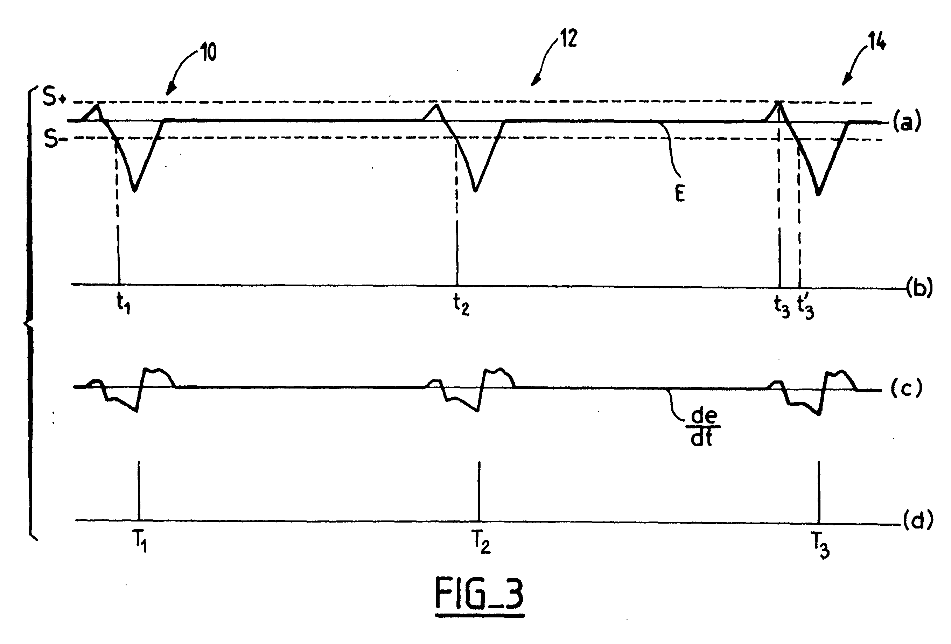

La figure 3 illustre sous forme comparative les avantages procurés par cette manière de procéder selon l'invention. Les chronogrammes (a) et (b) de la figure 3 correspondent à ceux de la figure 1, et les chronogrammes (c) et (d) de la figure 3 à ceux de la figure 2, pour trois cycles cardiaques successifs.

Comme on peut le constater, même si la forme d'onde varie légèrement d'un cycle au suivant, les intervalles I'₁ = T₂-T₁ , I'₂ = T₃-T₂, etc. présenteront une excellente stabilité, à la différence des intervalles I₁ = t₂-t₁, I₂ = t₃-t₂, etc. évalués par les systèmes à seuil de l'art antérieur. L'instabilité mesurée sera donc intrinsèque au rythme et non plus due au système de mesure.

Globalement, le système de détection de l'invention va donc fournir pour chaque onde de dépolarisation détectée, deux informations, à savoir :
- un top de détection, à l'instant t sur le chronogramme (b), informant de façon rapide et sensible le stimulateur qu'une dépolarisation spontanée est survenue ; et
- un top de synchronisation, à l'instant T sur le chronogramme (d), délivré secondairement, mais de façon plus spécifique, à des fins de mesure de séquencement temporel entre les différentes ondes de l'activité cardiaque.

## Revendications

1. Un dispositif médical implantable actif, notamment un stimulateur cardiaque, défibrillateur et/ou cardioverteur, comprenant des moyens de détection des dépolarisations spontanées (10, 12, 14) du myocarde, ces moyens de détection comportant un moyen primaire de traitement d'un signal cardiaque (E) délivrant une information de survenue d'une dépolarisation,
les moyens de détection comportant en outre un moyen secondaire de traitement du signal cardiaque, qui:
- est mis en oeuvre conditionnellement en cas de survenue de dépolarisation détectée au cours d'un cycle cardiaque par le moyen primaire, et
- recherche, au cours de ce même cycle cardiaque, une caractéristique unique du signal cardiaque donnant une information temporelle (T₁, T₂, T₃) sur l'instant de survenue de cette dépolarisation,
dispositif **caractérisé en ce que** le moyen secondaire comporte un moyen de recherche d'un pic d'autocorrélation dans le signal cardiaque, l'instant de survenue de la dépolarisation étant déduit de la position temporelle de ce pic.

2. Le dispositif de la revendication 1, dans lequel la recherche de ladite caractéristique unique du signal cardiaque par le moyen secondaire est effectuée à l'intérieur d'une fenêtre d'analyse de durée limitée.

3. Le dispositif de la revendication 2, dans lequel la fenêtre d'analyse s'étend à partir de l'instant de survenue de la dépolarisation détectée par le moyen primaire.

4. Le dispositif de la revendication 2, dans lequel il est prévu en outre une fenêtre rétrograde supplémentaire, telle que la fenêtre résultante totale s'étende autour de l'instant de survenue de la dépolarisation détectée par le moyen primaire.

## Patentansprüche

1. Aktivierbare implantierbare medizinische Vorrichtung, insbesondere ein Herzschrittmacher, Defibrillator und/oder Kardiovertierer, umfassend Mittel zur Erfassung von spontanen Depolarisationen (10, 12, 14) des Herzmuskels, wobei die Erfassungsmittel ein primäres Mittel zur Verarbeitung eines Herzsignals (E) aufweisen, das eine Information des Auftretens einer Depolarisation liefert,
wobei das Mittel zur Erfassung ferner ein sekundäres Mittel zur Verarbeitung des Herzsignals aufweist, welches:
- abhängig vom Fall des Auftretens einer Depolarisation in Betrieb gesetzt wird, die im Verlauf eines Herzzyklus durch das primäre Mittel erfasst wird, und
- die im Verlauf des gleichen Herzzyklus ein einzelnes Merkmal des Herzsignals sucht, das eine Zeitinformation (T₁, T₂, T₃) über den Moment des Auftretens dieser Depolarisation liefert,
wobei die Vorrichtung **dadurch gekennzeichnet ist, dass** das zweite Mittel ein Mittel zum Suchen eines Autokorrelations-Peaks im Herzsignal aufweist, wobei der Moment des Auftretens der Depolarisation von der temporalen Position dieses Peaks abgeleitet wird.

2. Vorrichtung nach Anspruch 1, in welcher die Suche des einzelnen Merkmals des Herzsignals durch das sekundäre Mittel veranlasst wird innerhalb eines Analysefensters von begrenzter Dauer.

3. Vorrichtung nach Anspruch 2, in welcher das Analysefenster sich ausgehend von dem durch das primäre Mittel erfassten Moment des Auftretens der Depolarisation erstreckt.

4. Vorrichtung nach Anspruch 2, in welcher ferner ein zusätzliches retrogrades Fenster vorgesehen ist, so dass sich das sich ergebende Gesamtfenster um den Moment des Auftretens der Depolarisation erstreckt, die durch das primäre Mittel erfasst wurde.

## Claims

1. An active implantable medical device, in particular a cardiac pacemaker, defibrillator and/or cardioverter, comprising means for sensing spontaneous depolarisations (10, 12, 14) of the myocardium, said sensing means including a primary means for processing a cardiac signal (E) issuing an information upon the occurrence of a depolarisation,
the sensing means further including a secondary means for processing the cardiac signal which:
- is conditionally operated in the case of occurrence of a depolarisation sensed during a cardiac cycle by the primary means, and
- searches, in the course of this very cardiac cycle, for a unique feature of the cardiac cycle which provides a temporal information (T₁, T₂, T₃) about the time of occurrence of said depolarization,
said device being **characterised in that** the secondary means includes a means for searching for an auto-correlation peak in the cardiac signal, the time of occurrence of the depolarisation being derived from said auto-correlation peak.

2. The device of claim 1, wherein the search for the unique feature of the cardiac signal by the secondary means is performed during a time window of limited duration.

3. The device of claim 2, wherein said time window spreads from the time of occurrence of the depolarisation detected by the primary means.

4. The device of claim 2, wherein there is provided an additional backwards window such that the full resulting window spreads around the time of occurrence of the depolarisation detected by the primary means.
